# EUROPÄISCHE PATENTSCHRIFT

(11) **EP 1 340 977 B1**
(45) Veröffentlichungstag und Bekanntmachung des Hinweises auf die Patenterteilung: **28.09.2005**
(21) Anmeldenummer: 03003562.0
(22) Anmeldetag: 17.02.2003
(51) Int. Cl.: G01N 33/44, B29C 44/02, G01N 1/04, G01N 1/28

(54) **Screeningverfahren zur Herstellung und Charakterisierung von Polyurethanschaumstoffen**
Screening method for making and characterising polyurethane foams
Méthode de screening pour la fabrication et la caractérisation de mousses de polyuréthane

(30) Priorität: 28.02.2002 DE 10208952
(43) Veröffentlichungstag der Anmeldung: 03.09.2003
(73) Patentinhaber: Bayer MaterialScience AG, 51368 Leverkusen (DE)
(72) Erfinder: Wagner, Joachim, Dr., 51061 Köln (DE); Jähn, Peter, 51375 Leverkusen (DE); Kusan-Bindels, Jacqueline, Dr., 41468 Neuss (DE); Ulbrich, Dagmar, Dr., 51065 Köln (DE); Albach, Rolf, Dr., 51061 Köln (DE); Kirschbaum, Hermann-Josef, 50169 Kerpen (DE)

(56) Entgegenhaltungen:
- EP-A- 1 176 413
- WO-A-00/70323
- DE-A- 2 835 038
- DE-A- 19 730 891
- DE-A- 19 743 590
- DE-A- 19 810 092
- FR-A- 2 447 030
- FR-A- 2 804 898
- US-A- 4 272 049
- PATENT ABSTRACTS OF JAPAN vol. 012, no. 424 (M-761), 10. November 1988 (1988-11-10) & JP 63 160809 A (HUMAN IND CORP), 4. Juli 1988 (1988-07-04)

## Beschreibung

Gegenstand der Erfindung ist ein Verfahren zur schnellen und effizienten Herstellung und Untersuchung von Rezepturen für Polyurethanschäume mit kleinen Substanzmengen.

Im Allgemeinen wird ein Screening von PUR-Schaumrezepturen per Hand durchgeführt, d.h. es werden Laborpäckchen mit 200 bis 300g Schaum hergestellt, wobei alle Zutaten manuell eingewogen, mit einem Standrührer vermischt, und die Mischung in Papierpäckchen eingegossen wird. Nachteile dieses manuellen Screenings sind niedriger Durchsatz von maximal 15 Päckchen pro Tag und Mitarbeiter, schlechte Reproduzierbarkeit durch nicht dokumentierte Fehler/Abweichungen bei Einwaagen, Rührzeiten, Rührerdrehzahl etc. und eine mühsame Bestimmung von Reaktionsparametern wie Startzeit, Steigzeit, Abbindezeit und Klebfreizeit per Hand.

Für die physikalische Prüfung von PUR-Schäumen ist problematisch, dass eine Entnahme mehrer identischer Probenkörper nach DIN (Probengröße mindestens 125 cm³) auf Grund von Fließwegphänomenen und Rohdichteschwankungen (bis zu 10 %) und der begrenzten Probenmenge aus einem identischen Ansatz nicht möglich ist und dass auf Grund zerstörender Prüftechniken der Probenkörper oft auch nur für eine einzige Messung verwendet werden kann, weshalb man häufig auf die Verwendung mehrerer möglichst identischer Päckchen angewiesen ist, die jedoch oft voneinander abweichende Eigenschaften, z.B. unterschiedliche Rohdichten oder Offenzelligkeiten aufweisen können. Um eine Alterung der Proben durch Zellgasaustausch zu vermeiden oder zu standardisieren, müssen vor einer Untersuchung der Proben definierte Lagerzeiten eingehalten werden.

Es wurde nun ein Verfahren entwickelt, mit dem sich der Zeit- und Materialaufwand für das Screening von Rezepturen für Polyurethanschäume stark verringern und zudem die Verlässlichkeit und Reproduzierbarkeit der erhaltenen Messdaten vergrößern lässt.

Dabei werden 10 bis 50 g, vorzugsweise 10 bis 20 g, besonders bevorzugt 16 g der jeweiligen Polyurethanformulierung in verschließbare Behälter mit einem Volumen von 10 bis 1000 ml, bevorzugt 125 bis 500 ml, die aus einem gasdichten Material bestehen (beispielsweise Polypropylen (PP), Polyethylen (PE), oder Polystyrol (PS)), eingebracht. Bevorzugt werden hierfür die einzelnen Komponenten in den Behälter eingewogen und darin vermischt. Es kann aber auch die fertige Reaktionsmischung in die Behälter eingefüllt werden. Die Behälter haben bevorzugt die Form zylindrischer Becher mit möglichst flachem Boden. Das Einwiegen erfolgt bevorzugt mittels einer automatisierten, computergesteuerten Dosierstation, wie sie in der Deutschen Patentanmeldung 101 59 272.8 beschrieben ist. Die Behälter werden nach dem Einwiegen oder nach dem Ende des Schäumvorgangs mit einem Deckel aus dem gleichen Material gasdicht verschlossen, wodurch ein Austausch des Zellgases im PUR-Schaum und ein Eindringen von Luft verhindert wird. Da so die Alterung der Proben unterdrückt wird, werden auch nach längerer Lagerzeit von ca. 6 Monaten bei physikalischen Untersuchungen keine verfälschten Werte erhalten.

Bevorzugt werden die Schäumbehälter aus transparentem oder transluzentem Material gefertigt, damit während des Schäumvorgangs optische Messungen wichtiger Parameter wie Steighöhe, Startzeit oder Steigzeit möglich sind. Diese können beispielsweise durch eine Videokamera mit angeschlossener Bildauswertung erfolgen, wie z.B. in L. Lefebvre and R. Keunings: "Mathematical Modelling and Computer Simulation of the Flow of Chemically-Reacting Polymeric Foams", in M.Cross et al. (Hrsg): "Mathematical Modelling in Materials Processing", Oxford Univ. Press, 1991, S. 399-417 beschrieben.

Zur Herstellung von Probenkörpern werden die schaumgefüllten Behälter in definiertem Abstand vom Behälterboden parallel zur Grundfläche (bzw. orthogonal zur Mantelfläche) in Scheiben definierter Dicke zerteilt, wobei darauf zu achten ist, dass auf der Oberfläche keine Riefen und Streifen entstehen. Dies kann z.B. mit einer Bandsäge mit schmalem, tiefem Sägeband mit möglichst hoher Zahnanzahl pro cm und geschränkten Zähnen geschehen. Bewährt haben sich Probendicken von etwa 10 mm bis 30 mm, bevorzugt 10 mm bis 20 mm und besonders bevorzugt 10 mm. Wichtig ist, dass die Probenkörper glatte Oberflächen aufweisen, um den Einfluss angeschnittener Zellen auf die Messwerte zu minimieren. Auch muss die Probenpräparationen so ausgeführt werden, dass die Zellstege glatt zerschnitten werden und nicht bei der Präparation wegbrechen und dabei tiefere Zellschichten zerstören.

Aus einer Scheibe werden Probenkörper für die physikalische Untersuchung präpariert, indem jeweils in gleichem Abstand zum Mittelpunkt (oder zum Rand) der Scheibe mehrere, bevorzugt 2 bis 8, besonders bevorzugt 4 Zylinder mit einem Hohlbohrer ausgebohrt werden. Damit sind konzentrisch auftretende Rohdichteänderungen in allen Zylindern identisch. Bewährt hat sich ein Zylinderdurchmesser von 10 bis 20 mm, bevorzugt 10 mm. Wichtig ist, dass die Zylinder schneidend gebohrt und nicht gestanzt werden, da sonst die Oberfläche der Probe beeinträchtigt wird (siehe oben).

Im nächsten Schritt werden die Abmessungen (Höhe, Durchmesser) und das Gewicht der Zylinder überprüft, um sicherzustellen, dass die Rohdichten der Probenkörper innerhalb einer Toleranz von 2 %, bevorzugt unter 1 % identisch sind. Ist dies der Fall, werden die Probenkörper zur Bestimmung physikalischer Daten des zu prüfenden Schaums verwendet. Es lassen sich beispielsweise Offenzelligkeit, Druckfestigkeit, Wärmeleitzahl, Dimensionsstabilität bei Warm- oder Kaltalterung mit konventionellen Messgeräten und/oder automatisierter Datenerfassung bestimmen. Entscheidender Vorteil des erfindungsgemäßen Verfahrens ist, dass sich auf Grund der Präparation von Probenkörpern mit identischen Eigenschaften mehrere unterschiedliche Parameter aus einem Polymer-Ansatz simultan und parallel bestimmen lassen.

Mehrere Scheiben unterschiedlicher Rohdichte können untersucht werden, um dadurch eine Abhängigkeit der physikalischen Eigenschaften von der Rohdichte bei sonst identischer PUR-Rezeptur zu ermitteln.

### Beispiel

Zur Herstellung einer Polyolformulierung wurden 5,81 g eines Polypropoxyethers mit einer Hydroxylzahl von 460 mg KOH/g, einer Funktionalität von 3 und einem zahlenmittleren Molgewicht von 370 g/mol, 0,44 g Tris(1-chlor-2-propyl)phosphat, 0,09 g eines Polyethersiloxan-Stabilisators (Niax® SR242, OSI Specialties Germany GmbH), 0,12 g Wasser 0,06 g Dimethylcyclohexylamin als Katalysator in einen 15 cm hohen zylindrischen PE-Synthesebehälter mit einem Durchmesser von 6,9 cm, d.h. mit einem Volumen von 500ml, dosiert und vermischt. Anschließend wurden in die so hergestellte Polyolformulierung, 0,61 g Cyclopentan als Treibmittel und 8,87 g Diphenylmethyldiisocyanat (MDI) mit einem Anteil von 31 Gew.-% NCO-Gruppen, 38 Gew.-% 4,4'-, 2,4'- und 2,2'-Isomeren und 62 Gew.-% mehrkerniger MDI-Oligomere eingemischt, woraufhin die Schäumreaktion zur einem Polyurethan-Hartschaum sichtbar einsetzte; der PE-Synthesebehälter wurde nicht verschlossen.

Der aus 16 g Reaktionsgemisch hergestellte Schaum wurde gemeinsam mit seinem PE-Synthesebehälter auf einer Bandsäge (Typ BS 400 E, Fried. Aug. Arnz "Flott" GmbH & Co., D-42857 Remscheid, Sägeblatt mit einer Dicke von 0,7 mm, einer Tiefe von 6,25 mm und 10 Zähnen/cm) zersägt; der untere Teil des Schaumzylinders mit einer Dicke von 2 cm wurde verworfen. Das darüber stehende Material wurde in 1 cm dicke Scheiben zerteilt.

Aus den Scheiben in den Höhen 3 cm bis 10 cm (Unterkante) wurden im Abstand von 1,8 cm zum Mittelpunkt der Scheibe jeweils 5 Zylinder mit einem Durchmesser von 1 cm geschnitten. Hierzu wurde ein Bohrzylinder mit einem Innendurchmesser von 1 cm, ähnlich einem Korkbohrer, jedoch aus gehärtetem Edelstahl mit integriertem Auswerfer, eingesetzt, um den Schaum beim Herausdrücken nicht zu komprimieren. Durchmesser und Dicke der Zylinder sowie deren Gewicht wurden gemessen und daraus die Rohdichte der Zylinder berechnet. An den innerhalb der Toleranz identischen Zylindern wurden Offenzelligkeit und Druckfestigkeit des Schaums bestimmt.

Die Offenzelligkeit des Schaums wurde über Gasverdrängungsverfahren (Pyknometrie) bestimmt. Gemessen wird hierbei, welches Volumen der Probe bei geringen Gasdrücken eines Edelgases von außen erreicht werden kann im Verhältnis zum geometrischen Volumen der Probe; die Bestimmung der Offenzelligkeit erfolgte ohne Korrektur für bei der Probenpräparation angeschnittene Oberflächen. Die Druckfestigkeit des Schaums wurde über die Bestimmung von Druck-Verformungskurven ermittelt (Messen der Kraft pro Probenfläche bei einer Deformation von 15 %).

Nachfolgende Tabelle gibt die einzelnen Messwerte wieder. Um zu demonstrieren, dass innerhalb der Toleranz identische Probenkörper gleiche Messwerte liefern, wurden jeweils an allen Probekörpern Druckfestigkeit und Offenzelligkeit bestimmt.

| Probenscheibe | Rohdichte | | Druckfestigkeit | | Offenzelligkeit | |
|---|---|---|---|---|---|---|
| | Mittelwert | Standard-abw. | Mittelwert | Standard-abw. | Mittelwert | Standard-abw. |
| | [g/l] | [g/l] | [MPa] | [MPa] | [%] | [%] |
| 3 cm | 36,0 | 0,32 | 0,30 | 0,006 | 26 | 3,1 |
| 4 cm | 33,9 | 0,34 | 0,29 | 0,015 | 25 | 2,8 |
| 5 cm | 33,8 | 0,32 | 0,28 | 0,008 | 29 | 3,4 |
| 6 cm | 32,9 | 0,30 | 0,29 | 0,006 | 30 | 2,0 |
| 7 cm | 32,5 | 0,29 | 0,27 | 0,015 | 32 | 1,6 |
| 8 cm | 32,0 | 0,31 | 0,24 | 0,018 | 31 | 1,4 |
| 9 cm | 31,2 | 0,22 | 0,23 | 0,014 | 30 | 2,1 |

## Patentansprüche

1. Screening-Verfahren für Polyurethanschaumstoff-Rezepturen, bei dem
a) ein Polyurethanschaum-Reaktionsgemisch in einen Behälter aus gasdichtem Material eingebracht wird,
b) die Reaktionsmischung in dem gegebenenfalls verschlossenen Behälter aufschäumen gelassen wird,
c) der Polyurethanschaum enthaltende Behälter parallel zur Grundfläche zerteilt wird, so dass mindesten eine Scheibe einer vorgegebenen Dicke erhalten wird,
d) aus einer solchen Scheibe mehrere zylindrische Probenkörper mit innerhalb eines Toleranzbereichs identischen Abmessungen und identischer Rohdichte entnommen werden, und
e) diese Probenkörper zur parallelen Messung physikalischer Eigenschaften des Polyurethanschaums verwendet werden.

## Claims

1. Screening process for polyurethane foam material formulations, in which
a) a polyurethane foam reaction mixture is introduced into a container made of gas-tight material,
b) the reaction mixture is allowed to foam in the optionally sealed container,
c) the container containing polyurethane foam is divided in a manner parallel to the base area, such that at least one disk of a predetermined thickness is obtained,
d) a plurality of cylindrical sample bodies having dimensions which are identical to within a range of tolerance and having identical density are removed from such a disk, and
e) these sample bodies are used for the parallel measurement of physical properties of the polyurethane foam.

## Revendications

1. Méthode de screening pour les formulations de mousses de polyuréthane, dans laquelle :
a) on introduit un mélange réactionnel de mousse de polyuréthane dans un récipient en matériau étanche aux gaz,
b) on fait s'expanser le mélange réactionnel dans le récipient, le cas échéant fermé,
c) on divise le récipient contenant la mousse de polyuréthane parallèlement à la surface de base, de manière à obtenir au moins une tranche d'une épaisseur prédéterminée,
d) on prélève, d'un telle tranche, plusieurs éprouvettes cylindriques présentant des dimensions identiques et une masse volumique identique à l'intérieur d'un domaine de tolérances et
c) on utilise ces éprouvettes pour la mesure parallèle des propriétés physiques de la mousse de polyuréthane.
